# EUROPEAN PATENT APPLICATION

(11) **EP 4 371 465 A1**
(43) Date of publication of application: **22.05.2024**
(21) Application number: 22383109.0
(22) Date of filing: 16.11.2022
(51) Int. Cl.: A61B 1/012, A47L 15/13, A61B 1/12, B08B 3/12, A61B 90/70

(54) **REPROCESSING METHOD FOR REPROCESSING A MEDICAL INSTRUMENT FOR ROBOTIC SURGERY AFTER ITS USE**

(71) Applicant: Antonio Matachana S.A., 08018 Barcelona (ES)
(72) Inventor: Matachana Aramburu, Manuel, Barcelona (ES); Alonso Magadán, Marino, Barcelona (ES); Lorenzo Marfil, Elena, Barcelona (ES); Carreras Sangra, Nelson, Barcelona (ES); Hernández Yelo, Enrique, Barcelona (ES)
(74) Representative: Curell Suñol S.L.P.

(57) **Abstract**

The invention relates to a reprocessing method (1) for reprocessing a medical instrument (11) for robotic surgery after its use. The medical instrument (11) comprises a proximal end (27) and a distal end (29), a lumen (31) extending between said proximal and distal ends (27, 29) and an articulated surgical head (33) at said distal end (29).The method comprises the following steps: a pre-cleaning step (3) for removing a primary residue, a cleaning and disinfecting step (5) for cleaning and disinfecting said medical instrument (11) after said pre-cleaning step (3), and a sterilizing step (7) for sterilizing said medical instrument (11) after said cleaning and disinfecting step (5). In the pre-cleaning step (3), said medical instrument (11) is arranged in a tank (13) filled with a first cleaning liquid, and the medical instrument (11) is in fluid communication with pumping means (15), for circulating said first cleaning liquid (47) through said lumen (31). Furthermore, during said pre-cleaning step (3) an ultrasound effect generated by an ultrasound generator device (17) is further applied.

## Description

### Field of the invention

The invention relates to a reprocessing method for reprocessing a medical instrument for robotic surgery after its use, said medical instrument comprising a proximal end and a distal end, a lumen extending through said medical instrument between said proximal and distal ends and an articulated surgical head provided at said distal end.

### State of the art

Medical instruments for robotic surgery comprising a lumen extending through the medical instrument between a proximal end and a distal end and with an articulated surgical head provided at the distal end are known in the art. These surgical tools are directed to be introduced into the human body through incisions therethrough to carry out the surgical operations, e.g. such as clamping, grasping, clip application, cutting, suctioning, sewing, cauterizing or other operations directed to complete a surgical intervention.

An example of these medical instruments used in robotic surgical systems is the medical instruments of robotic surgical systems such as Da Vinci Surgical System of the company Intuitive Surgical. This system comprises interactive robotic instruments that are remotely controlled by a surgeon. There exist several types of instruments that can be used according to the type of medical procedure that must be performed. Several of these instruments have a lumen and an articulated head at its distal end that, when in contact with a patient's body, gets dirty. Therefore, once the procedure has been finished, they must be cleaned so soil, (mainly body tissue, blood, proteins, and some potential pathogens, or the like) is removed from them for later reuse in further patients. It is of outmost importance that the hygienic level of these instruments for robotic surgery, and particularly of their lumens, reaches certain predetermined levels. All the medical instruments for robotic surgery that must be reused could be a vector of infections in later patients that must be treated in case any biological residue remains in the medical instrument.

Currently, these instruments for robotic surgery are subjected to a predetermined reprocessing method prescribed by the manufacturer of the robotic system. This prescribed method comprises a pre-cleaning step, a cleaning and disinfecting step, and final a sterilizing step. The main problem of this reprocessing method is that to guarantee a secure hygienic condition, the medical instrument must be thoroughly reprocessed. This is thus a slow process, mainly because the pre-cleaning step consisting in a thorough rinsing of the lumen of the medical instrument is performed manually with the assistance of a brush. More precisely, a person pre-cleans the instrument by hand during long periods of time of more that approximately 60 minutes. The pre-cleaning step is to be carried out according to the predetermined cleaning instructions provided by the manufacturer, known in the art as IFUs (Instructions For Use). This pre-cleaning step has the object of removing from the outer or inner surfaces of the medical instrument of any remain of blood, tissues, fat, or other human or animal body parts stuck to the surfaces. Once this pre-cleaning step has been performed, the medical instrument is subjected to the cleaning and disinfecting step, and sterilizing step.

Furthermore, a drawback of this robotic surgical systems is that they have a relevant cost of implementation and use. Therefore, it is convenient that the systems can be in operation as much time as possible. One of the bottlenecks in this case is the reprocessing of the instruments of the system.

### Summary of the invention

It is an object of the invention to propose an improved reprocessing method for reprocessing a medical instrument for robotic surgery after its use. The method according to the invention must be more efficient in terms of time and of hygienic final conditions of the medical instrument. This purpose is achieved by a method for reprocessing a medical instrument for robotic surgery after its use, said medical instrument comprising a proximal end and a distal end, a lumen extending through said medical instrument between said proximal and distal ends and an articulated surgical head provided at said distal end, of the type indicated at the beginning, characterized in that the method comprises the following steps:
[i] a pre-cleaning step for removing a primary residue from said medical instrument,
[ii] a cleaning and disinfecting step for cleaning and disinfecting said medical instrument after said pre-cleaning step, and
[iii] a sterilizing step for sterilizing said medical instrument after said cleaning and disinfecting step,

in that in said pre-cleaning step, said medical instrument is arranged in a tank filled with a first cleaning liquid, and one end among said proximal and distal ends of said medical instrument being in fluid communication with pumping means, such that during said pre-cleaning step said pumping means correspondingly circulates said first cleaning liquid through said lumen, thereby cleaning said medical instrument, and in that during said pre-cleaning step an ultrasound effect generated by an ultrasound generator device is further applied to remove said primary residues.

The use of ultrasounds together with the circulation of the first cleaning liquid driven by the pumping means reduces the pre-cleaning step time considerably. Soil can be attached to any surface of the medical instruments, that is not only the lumen, but also the articulated head the outer surface of the instrument or any other part that has been in contact with the patient.

The pumping means can work either by suctioning of by pumping or even in both directions in the same pre-cleaning step. Moreover, as the proposed pre-cleaning step removes more soil, that is the primary residue, from the medical instrument than a manual pre-cleaning technique as normally indicated in the IFUs of medical instruments for robotic surgery comprising lumens through which several mechanical parts of the mechanical instruments are guided.

It has been observed that thanks to this improved pre-cleaning step, a synergetic effect occurs, since the time of the following cleaning and disinfecting steps can also be reduced. Although the sterilizing step remains the same, there is a noticeable reduction of the overall reprocessing time. However, all this shortening of the reprocessing time, opposite to what it could be expected, does not lead only to the good results on the hygienic level of the instruments reprocessed, but even to higher results in terms of cleanliness. All in all, the reprocessing procedure, leads to reduced standstill times of the medical instruments thus allowing to shortening the time required for the tools to be ready to be in ready for use again.

The invention is thus directed to the reprocessing of those medical instruments for robotic surgery conceived to be reused several times, before their final disposal.

The invention further includes a number of preferred features that are object of the dependent claims and the utility of which will be highlighted hereinafter in the detailed description of an embodiment of the invention.

Preferably, said method further comprises a filtering step and said pumping means forms a closed loop fluid system such that said first cleaning liquid recirculates between said tank and said pumping means, said cleaning liquid being subjected to said filtering step before entering anew said pumping means. Therefore, there is no need to constantly feed fresh first cleaning liquid to the tank. Therefore, a more economical and sustainable reprocessing method is achieved. The filtering step is carried out by interposing a filter in the hydraulic circuit to which the medical instruments is coupled to.

Preferably, the ultrasound generator device operates at a frequency comprised between 25 kHz and 45 kHz, more preferably at a frequency comprised between 35 kHz and 40 kHz. This frequency range has been found to be the optimal range for removing first primary residues such as body tissue, blood, proteins, and some potential pathogens, or the like from the medical instrument.

Even more preferably, in said pre-cleaning step said ultrasound generator device generates ultrasonic waves during a period comprised between 5 to 50 minutes and preferably between 10 and 35 minutes. This period ensures that the medical instrument is properly cleaned, and that the cleaning step can be noticeably shortened in comparison to the state-of-the-art methods.

In an embodiment seeking to reduce the energy consumption, said first cleaning liquid circulation through said lumen and said ultrasound effect generation take place in at least partly overlapped manner. In this case the ultrasound could be interrupted during the first liquid circulation or else, the ultrasound can be applied all the time, and the first liquid circulation can be intermittently stopped. Also, the periods of time of overlap can have a variable duration, depending on whether the pre-cleaning process is starting or ending.

Alternatively, to accelerate the pre-cleaning step, preferably said first cleaning liquid circulation through said lumen and said ultrasound effect generation are completely overlapped.

In another embodiment, said ultrasound generator device operates at a variable frequency to obtain different cleaning profiles, depending on the tidiness of the surgical tool and the instant of the pre-cleaning step.

Preferably, said pumping means applies a volumetric flowrate of first cleaning liquid circulating through said lumen comprised between 200 and 500 ml/min and preferably between 300 and 400 ml/min. his pressure ensures an optimal first cleaning liquid recirculation and an optimal cleaning of the medical instrument by drawing the most first primary residue.

Preferably, said first cleaning liquid of said pre-cleaning step is at a temperature comprised between 15 °C and 45 °C, preferably at a temperature comprised between 20 °C and 40 °C. This temperature range ensures that no vapours and aerosols that have impurities escape the tank, reducing the infection risk. Furthermore, it also avoids protein coagulation.

More preferably, during said pre-cleaning step more than one medical instrument can be cleaned at a time. This allows cleaning several medical instruments at the same time, hence, reducing the pre-cleaning time if several medical instruments must be cleaned. Thanks to this the either the standstill times of the medical instruments can be reduced or else, less medical instruments are required in stock. It is possible to have a medical instrument support with the corresponding coupling means for each medical instrument. This support can be pivotally mounted to facilitate the immersion and extraction of the instruments from the pre-cleaning tank.

Preferably, in said a cleaning and disinfecting step, said medical instrument to be cleaned and disinfected is arranged in a cleaning and disinfecting device such that one end among said proximal and distal ends of said medical instrument is in fluid communication with second pumping means, such that during said cleaning and disinfecting step, said second pumping means correspondingly circulates said second cleaning liquid through said lumen, thereby cleaning said medical instrument. Again the circulation of a second cleaning liquid through the lumen of the medical instrument provides for a more thorough cleaning of the inside soil of the medical instrument.

Preferably, said pre-cleaning step is performed during a period of time comprised between 20 and 50 minutes, more preferably between 25 and 45 minutes, and even more preferably between 30 and 38 minutes. This time has been found to be the optimal time for pre-cleaning the medical instrument.

More preferably, said cleaning and disinfecting step is performed during a period of time comprised between 5 and 25 minutes, more preferably between 5 and 15 minutes, and even more preferably for 10 minutes. This time has been found to be the optimal time for cleaning and disinfecting the medical instrument without compromising the sanitary conditions for reuse.

In a preferred embodiment seeking to reduce the accumulation of pathogens to the minimum before entering the sterilizing step, said cleaning and disinfecting step said second cleaning liquid is at a temperature between 80 and 95 °C and preferably between 85 and 93 °C.

In a preferred embodiment said sterilizing step consists in one among sterilization by water saturated steam, or low temperature sterilization technologies such as vaporized hydrogen peroxide, low temperature steam and formaldehyde, and ethylene oxide. Again, the optimum reprocessing results are obtained by any of these methods.

Even more preferably, after pre-cleaning and the cleaning steps have been performed, an amount of protein per sample should be < 100 µg per medical device according to Guideline Hygiene Requirements for the Reprocessing of Medical Devices (RKI)/(BfArM) 2012 - 55:1244-131. Therefore, once the reprocessing method has been completed, the medical instrument meets the requirements to be reused in secure sanitary conditions.

To achieve the best pre-cleaning step results, preferably, said first cleaning liquid comprises a mixture of decalcified water and detergent, said detergent having an enzymatic nature and a pH range comprised between 8,5 and 11, or preferably between 10 and 10.8.

Also to achieve the best cleaning and disinfecting step results, preferably, said second cleaning liquid comprises a mixture of decalcified water and detergent, said detergent having an enzymatic nature and a pH range comprised between 8,5 and 11, or preferably between 10 and 10.8.

Likewise, the invention also includes other features of detail illustrated in the detailed description of an embodiment of the invention and in the accompanying figures.

### Brief description of the drawings

Further advantages and features of the invention will become apparent from the following description, in which, without any limiting character, preferred embodiments of the invention are disclosed, with reference to the accompanying drawings in which:
Figure 1 shows a scheme of a reprocessing method according to a first embodiment of the invention.
Figure 2 shows diagrammatically a surgery robot with a plurality of medical instruments for robotic surgery.
Figure 3 shows diagrammatically a medical instrument for robotic surgery to be cleaned with the method according to the invention.
Figure 4 shows one after the other, the pre-cleaning device for removing a primary residue from said medical instrument, the cleaning and disinfecting device for cleaning and disinfecting the medical instrument after the pre-cleaning step, and the sterilizing device for performing the reprocessing method of Figure 1.
Figure 5 shows diagrammatically the main components of the pre-cleaning device Figure 4.
Figure 6 shows a comparison graph between the protein Acceptance Level per medical device according to the Guideline Hygiene Requirements for the Reprocessing of Medical Devices of the Rober Koch Institut (RKI)/(BfArM) 2012 - 55:1244-131 and the results obtained with the pre-cleaning and cleaning steps of the reprocessing method according to the invention.

### Detailed description of embodiments of the invention

The term *"reprocessing method"* refers to an overall cleaning process carried out on used medical instruments for robotic surgery to allow its safe sanitary reuse.

The *"reprocessing method"* according to the invention comprises at least a pre-cleaning step 3, a cleaning and disinfecting step 5, and a sterilizing step 7.

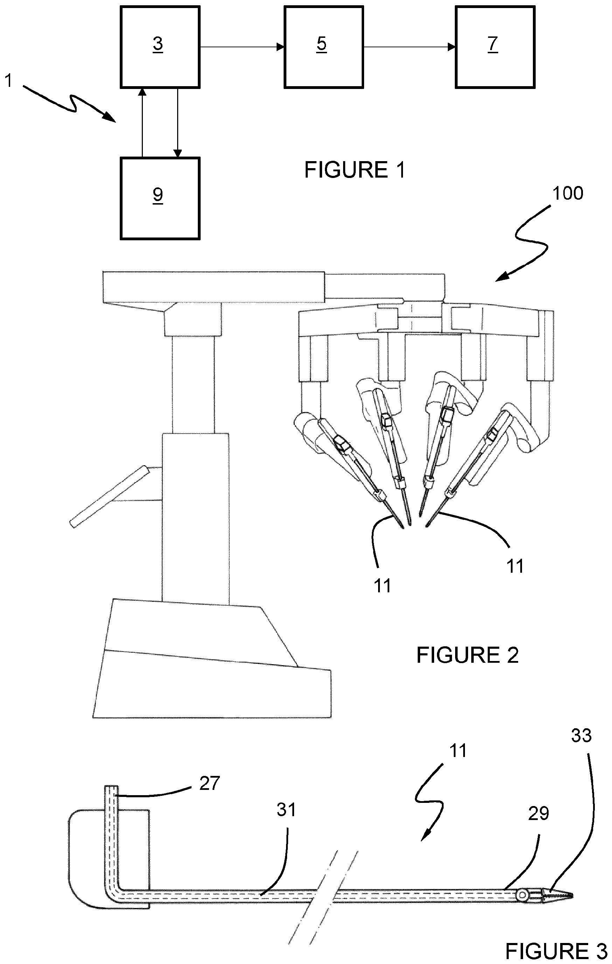

The invention refers to a reprocessing method 1 for reprocessing a medical instrument 11 for robotic surgery after its use. As shown in the diagrammatical Figure 3, the medical instrument 11 comprises a proximal end 27 and a distal end 29, a lumen 31 extending through the medical instrument 11 between the proximal and distal ends 27, 29 and an articulated surgical head 33 provided at the distal end 29. This kind of instruments 11 are used in robotic surgery systems 100 as the one shown in figure 2.

The high cost of these robotic surgery systems 100 requires that the stillstands of the robotic system 100 is as short as possible. On the other hand, the medical instruments 11 have also a relevant cost and for this reason they are intended be reused several times, after a corresponding reprocessing procedure. Finally, after several reprocessing cycles the live cycle of the medical instrument 11 is finished.

As already explained it is an object of the invention that the reprocessing method 1 is more efficient in terms of time and of hygienic final conditions of the medical instrument 11.

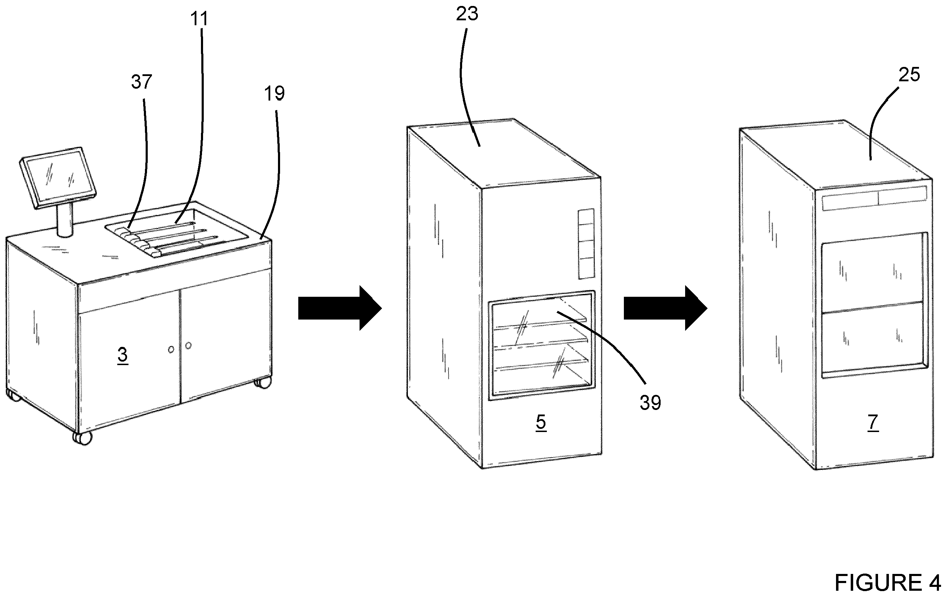

To this end, as shown in Figures 1 or 4, the method according to the invention comprises the following steps:
[i] a pre-cleaning step 3 for removing a primary residue from the medical instrument 11,
[ii] a cleaning and disinfecting step 5 for cleaning and disinfecting the medical instrument 11 after the pre-cleaning step 3, and
[iii] a sterilizing step 7 for sterilizing said medical instrument 11 after the cleaning and disinfecting step 5.

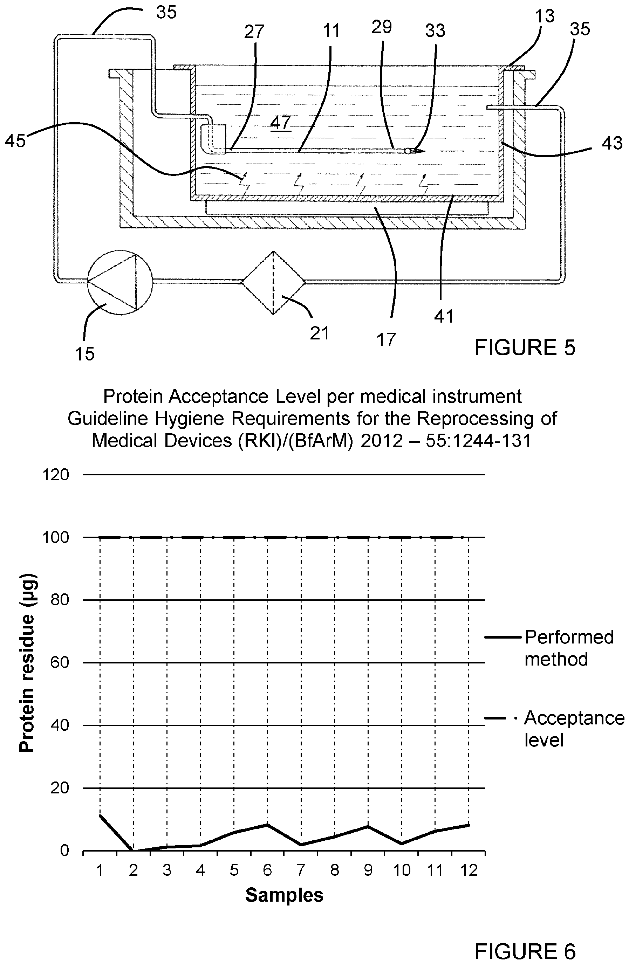

According to the invention in the pre-cleaning step 3, the medical instrument 11 is arranged in a tank 13 filled with a first cleaning liquid 47. To achieve the best pre-cleaning step results, preferably, the first cleaning liquid 47 comprises a mixture of decalcified water and detergent in an appropriate quantity of the later. The detergent has an enzymatic nature and a pH range comprised between 8,5 and 11, or preferably between 10 and 10.8. Once arranged in the tank 13 one end among the proximal and the distal ends 27, 29 of the medical instrument 11 is in fluid communication with pumping means 15. As it is apparent from Figure 5, in this embodiment, the proximal end 27 of the medical instrument 11 is coupled to a duct 35 connected to a hydraulic circuit comprising pumping means 15, such as a centrifugal pump, or similar device. Upstream of the pumping means 17, there is a filter 21 arranged, which at its time is again connected via the duct 35 to the other side of the tank 13. Therefore, in the preferred embodiment shown, the method further comprises a filtering step 9 and the pumping means 15 forms a closed loop fluid system such that the first cleaning liquid 47 recirculates between the tank 13 and the pumping means 15. The first cleaning liquid 47 is subjected to the filtering step 9 before entering anew the pumping means 15.

As it is apparent from Figure 5, the side of the duct 35 connecting the tank 13 and the filter 21 flows at the upper part of the tank 13. Although the duct 35 of the right side of the tank 13 could be arranged also at a lower level, it is preferable that it is placed at a higher position of the tank, since the first cleaning liquid 47 is cleaner at this upper part of the tank 13 than at the bottom 41 thereof.

During the pre-cleaning step 3 the pumping means 15 correspondingly circulates the first cleaning liquid 47 through the lumen 31, thereby cleaning the medical instrument 11. The first cleaning liquid 47 of the pre-cleaning step 3 is at a temperature comprised between 15 °C and 45 °C, and preferably at a temperature comprised between 20 °C and 40 °C. Also, it must be pointed out that the flow direction of the first cleaning liquid 47 is preferably in the direction from the proximal end 27 to the distal end 29 of the medical instrument 11. This tends to easier expel the residue of the distal end 29 towards the outside of the medical instrument 11. However, during the pre-cleaning step 3 the flow direction of the first cleaning liquid 47 can be also reversed. It is also possible that the first cleaning liquid 47 is alternatively circulated back and forth during the whole pre-cleaning step period during some periods of time.

Furthermore, to remove the primary residues contained in the medical instrument 11 and more particularly inside the lumen 31, during the pre-cleaning step 3 an ultrasound effect generated by an ultrasound generator device 17 is further applied to remove the primary residues.

According to the invention, in the pre-cleaning step 3, one or several medical instruments 11 can be attached to a tiltable support 37. In this case, Figure 4 shows how four medical instruments can be pre-cleaned at a time, thus reducing even more the time required for the overall reprocessing procedure.

Further, in the pre-cleaning step 3 the pumping means 15 applies a volumetric flowrate of first cleaning liquid 47 circulating through the lumen 31 comprised between 200 and 500 ml/min and preferably between 300 and 400 ml/min.

The ultrasound effect is generated by an ultrasound generator device 17, such as an ultrasonic transducer, generating ultrasonic waves 45. In this case, the ultrasound generator device 17 produces the ultrasonic waves 45 from the bottom of the tank 13 to the top thereof. However, the ultrasound generator device 17 or devices could also be arranged on other walls of the tank 13, such as the side walls 43. The ultrasonic waves 45 cause the first cleaning liquid 47 to vibrate, and through cavitation, destroy and separate the primary residues on the surface of the medical instrument 11 and especially in the lumen 31 thereof. This technique removes more soil than the manual technique commonly used in the art, because the ultrasonic waves 45 are more effective in peeling off blood, fat, muscle or similar by-products stuck to the inner wall of the lumen 31.

The ultrasound generator device 17 according to the invention operates at a frequency comprised between 25 kHz and 45 kHz during a period comprised between 5 to 50 minutes. However, more preferably the frequency is comprised between 35 kHz and 40 kHz and it is used during a period of time comprised between 10 and 35 minutes. In the pre-cleaning step 3, the first cleaning liquid 47 circulation through the lumen 31 and the ultrasound effect generation can take place in at least partly overlapped manner. However, it is also possible that the first cleaning liquid 47 circulation through the lumen 31 and said ultrasound effect generation are completely overlapped to each other. Finally, it is also possible that the ultrasound generator device 17 operates at a variable frequency.

Figure 4 shows a diagrammatical example of a pre-cleaning device 19 in which said pre-cleaning step 3 could be performed. As it can be seen in Figure 4, during said pre-cleaning step 3 more than one medical instrument 11 can be cleaned at the same time.

Preferably the pre-cleaning step 3 is performed during a period of time comprised between 20 and 50 minutes, more preferably between 25 and 45 minutes, and even more preferably between 30 and 38 minutes.

Once the pre-cleaning step 3 is finished, the medical instrument 11 is subjected to the cleaning and disinfecting step 5. In the invention it has been observed that the pre-cleaning step allows a shorter cleaning and disinfecting step 5, being it much quicker than in the equivalent state of the art step. Particularly, in the invention it is relevant the combined effect between the pre-cleaning and the cleaning steps. In other words, in terms of protein removal, the disinfection step through temperature, is little involved. Thanks to the combined effect the medical instrument 11 contains less soil that needs to be remove, making thus this second step much easier in a synergic manner between both steps.

To carry out the cleaning and disinfecting step 5, preferably the medical instrument 11 to be cleaned and disinfected is arranged in a cleaning and disinfecting device 23 such that one end among said proximal and distal ends 27, 29 of the medical instrument 11 is in fluid communication with second pumping means, not shown in figure 4. During the cleaning and disinfecting step 5 it is specially preferred that the second pumping means correspondingly circulates the second cleaning liquid through the lumen 31, via the corresponding coupling means adapted to the medical instrument to introduce the second cleaning liquid into the instrument's lumen 31, thereby cleaning the medical instrument 11. Circulating the second cleaning liquid directly through the lumen 31 under pressure assures, preferably in the direction from the proximal end 27 to the distal end 29 of the medical instrument 11, a more reliable result in the disinfection of the medical instrument 11. Again, the best cleaning and disinfecting step results are obtained when the second cleaning liquid comprises a mixture of decalcified water and detergent. The detergent in this case has an enzymatic nature and a pH range comprised between 8,5 and 11, or preferably between 10 and 10.8.

Again, in this cleaning and disinfecting step 5, several medical instruments 11 can be cleaned at a time. To this end, a rack 39 is provided in the cleaning and disinfecting device 23 with the corresponding coupling means each medical instrument 11.

Preferably in the cleaning and disinfecting step 5, in the cleaning and disinfecting step 5, the second cleaning liquid is at a temperature between 80 and 100 °C, and preferably between 85 and 93 °C. Furthermore, thanks to the previous pre-cleaning step the cleaning and disinfecting step 5 needs only to be performed during a period of time comprised between 5 and 25 minutes, more preferably between 5 and 15 minutes, and even more preferably for 10 minutes.

Finally, after the cleaning and disinfecting step 5 has been performed, the medical instrument 11 is subjected to a sterilizing step 7. The sterilizing step 7 consists in one among sterilization by water saturated steam, vaporized hydrogen peroxide, low temperature steam and formaldehyde and ethylene oxide. This sterilizing step 7 can be performed using different types of sterilizing devices 25 known in the art. As an example, in the case of sterilizing using a water saturated steam procedure, good sterilization results are obtained at a pressure and temperature conditions between 1 bar at 120 °C and 2,8 bar at 140 °C inside the sterilizing device 25, such as e.g. 2,3 bar at 138 °C inside the sterilizing device 25.

When the reprocessing method, and more particularly the pre-cleaning 3 and cleaning steps 5 according to the invention have been finished, the amount of protein per sample in the medical device is considered to be optimum when it is below 100 µg per medical device according to Guideline Hygiene Requirements for the Reprocessing of Medical Devices (RKI)/(BfArM) 2012 - 55:1244-131 (See Anforderungen an die Hygiene bei der Aufbereitung von Medizinprodukten"; Bundesgesundheitsbl 2012 - 55:1244-131*).* Therefore, the medical instrument 11 is considered to meet the hygiene requirements to be safely disinfected with temperature and later sterilized as explained before.

Thanks to the complete method according to the invention, the medical instrument 11 can be used again in a safe manner from a healthcare point of view, but with a noticeable time reduction of the reprocessing time, in comparison to the reprocessing methods known in the art.

### Analysis of the combined effect of the pre-cleaning and cleaning steps of the reprocessing method according to the invention:

A test was conducted to analyse the combined effect of the pre-cleaning and cleaning steps 3, 5 in the reprocessing method 1 according to the invention. To this end, a Trison Bandelin ultrasound bath and a Matachana LD500 Washing disinfector were used for the test. It was found that the combination of these two steps provides the required improvements over the prior art reprocessing methods thus leading to a more efficient reprocessing method in terms of time and of hygienic final conditions of the medical instrument.

Therefore, in this test, these two steps were tested in an isolated manner from the rest of the reprocessing method of the invention. Therefore, the disinfection step was isolated from the cleaning step for the preliminary tests to measure specifically the cleaning efficacy of the process. Neither the sterilization step was carried out in this case.

The test was conducted on Xi and Si 8mm instruments of Maryland Bipolar Forceps (MBF) of the Da Vinci robotic surgical system of the company Intuitive Surgical. This medical instrument has the smallest surface area. This smallest surface area is 279,6 cm², and it is deemed to be the worst scenario for a reprocessing method.

The medical instruments 11 to be tested were first inoculated with soil, using sheep blood with protamine sulphate at an amount of 100 - 1000 µg protein per instrument. Further the head 33 of the instruments was dipped into the test soil 5 seconds and the head articulated several times. Then, the instruments 11 were let dry inside a cabinet for 60 minutes before starting the test, such that the soil stuck to the walls of the instruments.

The test carried out comprised a first pre-cleaning step 3 for 35 minutes, followed by a cleaning step 5 performed twice, being each cleaning step 5 performed for a period of 5 minutes.

This cycle was repeated three times with 4 instruments, thus in total, 12 samples were obtained as shown in Figures 6 to 8.

Tables 1 to 3 show the residues of protein, that is not only the primary residue after the pre-cleaning step, but also the residue after the cleaning step after each cycle for each of the 12 tested medical instruments 11.

In the tables, the acronym M.I. in the tables stands for "Medical Instrument".

Further the acronyms S and T stand for the shaft and the tip of the medical instrument. These two sections of the medical instrument are deemed to be the worst-case scenario in term of soiling.

The column Abs refers to the absorbance column in the detection method based on the Lambert-Beer law. This defines the function of the ratio of the absorbance of the molecules (protein) at a specific wavelength to the amount of protein.

Regarding the µg/ml column shows the extreme values of the Lambert-Beer formula.

The protein residue column (µg in extraction volume) is the amount measured for both S and T that is finally added and corresponds to the total protein S+T of the medical instrument.

**Table 1:**

| **M.I. Batch** | **ID** | **Abs** | **µg/ml** | **Protein residue (µg in extraction volume)** | **Protein residue (µg in M.I.)** |
|---|---|---|---|---|---|
| **437** | 1T | 0,083 | 1,39 | 5,55 | 11,12 |
| | 1S | 0,063 | 0,93 | 5,57 | |
| **452** | 2T | 0,01 | 0 | 0 | 0,88 |
| | 2S | 0,029 | 0,15 | 0,88 | |
| **457** | 3T | 0,036 | 0,31 | 1,23 | 1,23 |
| | 3S | 0,022 | 0 | 0 | |
| **450** | C- T | 0,027 | 0,10 | 0,40 | 1,56 |
| | C- S | 0,031 | 0,19 | 1,16 | |

**Table 2:**

| **M.I. Batch** | **ID** | **Abs** | **µg/ml** | **Protein residue (µg in extraction volume)** | **Protein residue (µg in M.I.)** |
|---|---|---|---|---|---|
| **437** | 1T | 0,071 | 1,11 | 4,45 | 5,74 |
| | 1S | 0,032 | 0,22 | 1,30 | |
| **452** | 2T | 0,081 | 1,34 | 5,37 | 8,18 |
| | 2S | 0,043 | 0,47 | 2,81 | |
| **457** | 3T | 0,024 | 0,03 | 0,13 | 1,84 |
| | 3S | 0,035 | 0,28 | 1,71 | |
| **450** | C- T | 0,053 | 0,70 | 2,79 | 4,36 |
| | C- S | 0,034 | 0,26 | 1,57 | |

**Table 3:**

| **M.I. Batch** | **ID** | **Abs** | **µg/ml** | **Protein residue (µg in extraction volume)** | **Protein residue (µg in M.I.)** |
|---|---|---|---|---|---|
| **437** | 1T | 0,055 | 0,74 | 2,98 | 7,72 |
| | 1S | 0,057 | 0,79 | 4,74 | |
| **452** | 2T | 0,04 | 0,40 | 1,60 | 2,20 |
| | 2S | 0,027 | 0,10 | 0,61 | |
| **457** | 3T | 0,049 | 0,61 | 2,43 | 6,20 |
| | 3S | 0,05 | 0,63 | 3,78 | |
| **450** | C- T | 0,046 | 0,54 | 2,15 | 8,13 |
| | C- S | 0,066 | 1,00 | 5,98 | |

Table 4 shows a summary of the protein residues of each cycle, see columns 7 and 8 of tables 1 to 3.

**Table 4:**

| **1st Cycle** | **2nd Cycle** | **3rd Cycle** |
|---|---|---|
| **Protein residue (µg in M.I.)** | **Protein residue (µg in M.I.)** | **Protein residue (µg in M.I.)** |
| 11,12 | 5,74 | 7,72 |
| 0,88 | 8,18 | 2,20 |
| 1,15 | 1,84 | 6,20 |
| 1,56 | 4,36 | 8,13 |

The obtained protein residue results were compared with the following acceptance criteria (see table 5 and Figure 6). In Figure 6, a comparison between each acceptance level criterion and the results obtained after the previously explained testing method is shown. In particular, Figure 6, also shows how in the results obtained the amount of protein per medical instrument or sample is < 100 µg per medical device according to Guideline Hygiene Requirements for the Reprocessing of Medical Devices (RKI)/(BfArM) 2012 - 55:1244-131.

It is apparent that the combination of the pre-cleaning step 3 and the cleaning step alone of the reprocessing method according to the invention not only meets the acceptance criterion, but clearly improves the protein residues levels accepted. This leads to an overall improvement of the reprocessing method of the invention. In fact, as it is apparent from the previous tables and Figure 6, the protein residue results are almost zero.

To this surprising performance of the method of the invention, it must be added that the time used in the reprocessing method of the invention is clearly shorter than the methods of the state of the art as shown below. Below, as an example, a comparison between the state-of-the-art validated cycle for the pre-cleaning and cleaning and disinfecting steps of the medical instruments of a Da Vinci Robot Surgical System according to the IFUs of the manufacturer Intuitive Surgical and the most relevant parts of the reprocessing method according to the invention can be seen in Table 6.

**Table 6:**

| **Validated cycle** | | **Cycle tested according to the invention** | |
|---|---|---|---|
| Pre-cleaning (performed manually, one medical instrument at a time) | 60 min | Pre-cleaning (up to four devices at the same time) | 35 min |
| Cleaning and disinfecting | 60 min | Cleaning and disinfecting | 10 min |
| TOTAL | 120 min | TOTAL | 45 min |

As shown in Table 6, the validated pre-cleaning cycle of the state of the art is performed manually, i.e. a person manually cleans the medical instrument with the aid of a brush according to the IFUs of the manufacturer. This process must be performed following certain steps indicated by the manufacturer, and it takes 60 minutes to pre-clean each medical instrument. In this case, a 62,5% reduction in the reprocessing time has been achieved.

After this pre-cleaning step has been performed, the medical instrument is taken into an automated cleaning and disinfecting device, where it is cleaned and disinfected for 60 minutes.

Finally, even though it is not shown in table 6, there is a step of sterilizing the medical instrument. This last step of sterilizing the medical equipment lasts the same amount of time in both methods, i.e. the validated method of the state of the art and the method according to the invention.

However, with the method according to the invention, the overall reprocessing time, i.e. pre-cleaning, cleaning and disinfecting, and sterilizing, is considerably reduced. This is due to the fact that the pre-cleaning step 3 according to the invention uses an automated system that combines ultrasounds with a pressurized first cleaning liquid 47 circulating through the lumen 31 of the medical instrument. This method has been proven to remove a greater amount of soil (protein, some potential pathogens, microorganisms, body tissue and the like) from the medical instrument 11. Therefore, the following cleaning and disinfecting step 5 can be performed for a shorter period of time, as the medical instrument 11 contains less residues.

Furthermore, several medical instruments 11 can be pre-cleaned at the same time. As seen in the results obtained in table 6, the pre-cleaning step 3 was performed for 35 minutes and the cleaning and disinfecting step 5 was performed during 10 minutes, reducing, therefore, the overall reprocessing time.

## Claims

1. A reprocessing method (1) for reprocessing a medical instrument (11) for robotic surgery after its use, said medical instrument (11) comprising a proximal end (27) and a distal end (29), a lumen (31) extending through said medical instrument (11) between said proximal and distal ends (27, 29) and an articulated surgical head (33) provided at said distal end (29), **characterized in that** said method comprises the following steps:
[i] a pre-cleaning step (3) for removing a primary residue from said medical instrument (11),
[ii] a cleaning and disinfecting step (5) for cleaning and disinfecting said medical instrument (11) after said pre-cleaning step (3), and
[iii] a sterilizing step (7) for sterilizing said medical instrument (11) after said cleaning and disinfecting step (5),
**in that** in said pre-cleaning step (3), said medical instrument (11) is arranged in a tank (13) filled with a first cleaning liquid, and one end among said proximal and distal ends (27, 29) of said medical instrument (11) being in fluid communication with pumping means (15), such that during said pre-cleaning step (3) said pumping means (15) correspondingly circulates said first cleaning liquid (47) through said lumen (31), thereby cleaning said medical instrument (11), and **in that** during said pre-cleaning step (3) an ultrasound effect generated by an ultrasound generator device (17) is further applied to remove said primary residues.

2. The method according to claim 1, **characterized in that** it further comprises a filtering step (9) and said pumping means (15) forms a closed loop fluid system such that said first cleaning liquid (47) recirculates between said tank (13) and said pumping means (15), said cleaning liquid being subjected to said filtering step (9) before entering anew said pumping means (15).

3. The method according to any one of claims 1 or 2, **characterized in that** the ultrasound generator device (17) operates at a frequency comprised between 25 kHz and 45 kHz, more preferably at a frequency comprised between 35 kHz and 40 kHz.

4. The method according to any one of claims 1 to 3, **characterized in that** said ultrasound generator device (17) generates ultrasonic waves during a period comprised between 5 to 50 minutes and preferably between 10 and 35 minutes.

5. The method according to any one of claims 1 to 4, **characterized in that** said first cleaning liquid (47) circulation through said lumen (31) and said ultrasound effect generation take place in at least partly overlapped manner.

6. The method according to claim 5, **characterized in that** said first cleaning liquid (47) circulation through said lumen (31) and said ultrasound effect generation are completely overlapped.

7. The method according to any one of claims 3 to 6, **characterized in that** said ultrasound generator device (17) operates at a variable frequency.

8. The method according to any one of claims 1 to 7, **characterized in that** said pumping means (15) applies a volumetric flowrate of first cleaning liquid (47) circulating through said lumen (31) comprised between 200 and 500 ml/min and preferably between 300 and 400 ml/min.

9. The method according to any one of claims 1 to 8, **characterized in that** said first cleaning liquid (47) of said pre-cleaning step is at a temperature comprised between 15 °C and 45 °C, preferably at a temperature comprised between 20 °C and 40 °C.

10. The method according to any one of claims 1 to 9, **characterized in that** during said pre-cleaning step (3) more than one medical instrument (11) can be cleaned at the same time.

11. The method according to any one of claims 1 to 10, **characterized in that** said a cleaning and disinfecting step (5) said medical instrument (11) to be cleaned and disinfected is arranged in a cleaning and disinfecting device such that one end among said proximal and distal ends (27, 29) of said medical instrument (11) is in fluid communication with second pumping means, such that during said cleaning and disinfecting step (5) said second pumping means correspondingly circulates said second cleaning liquid through said lumen (31), thereby cleaning said medical instrument (11).

12. The method according to any one of claims 1 to 11, **characterized in that** said pre-cleaning step (3) is performed during a period of time comprised between 20 and 50 minutes, more preferably between 25 and 45 minutes, and even more preferably between 30 and 38 minutes.

13. The method according to any one of claims 1 to 12, **characterized in that** said cleaning and disinfecting step (5) is performed during a period of time comprised between 5 and 25 minutes, more preferably between 5 and 15 minutes, and even more preferably for 10 minutes.

14. The method according to any one of claims 1 to 13, **characterized in that** in said cleaning and disinfecting step (5), said second cleaning liquid is at a temperature between 80 and 100 °C, and preferably between 85 and 93 °C.

15. The method according to any one of claims 1 to 14, **characterized in that** said sterilizing step (7) consists in one among sterilization by water saturated steam, vaporized hydrogen peroxide, low temperature steam and formaldehyde and ethylene oxide.
